# EUROPÄISCHE PATENTSCHRIFT

(11) **EP 1 746 859 B1**
(45) Veröffentlichungstag und Bekanntmachung des Hinweises auf die Patenterteilung: **22.07.2009**
(21) Anmeldenummer: 06014535.6
(22) Anmeldetag: 13.07.2006
(51) Int. Cl.: H04R 25/00, A61B 5/12

(54) **Vorrichtung und Verfahren zum Konfigurieren eines Hörgerätes**
Apparatus and method for fitting a hearing aid
Dispositif et procédé d'adaptation de prothèse auditive

(30) Priorität: 23.07.2005 DE 102005034560; 21.12.2005 DE 102005061150
(43) Veröffentlichungstag der Anmeldung: 24.01.2007
(73) Patentinhaber: Kurz, Hans-Rainer, 26639 Wiesmoor (DE)
(72) Erfinder: Kurz, Hans-Rainer, 26639 Wiesmoor (DE)
(74) Vertreter: Jabbusch, Matthias

(56) Entgegenhaltungen:
- DE-A1- 3 205 685
- US-A1- 2004 204 921
- US-B1- 6 496 585
- AVERY AND C SUSSKIND W H: "Simple Bekesy-Type Audiometer for Clinical Use" IEEE TRANSACTIONS ON ACOUSTICS, SPEECH AND SIGNAL PROCESSING, IEEE INC. NEW YORK, US, Bd. ASSP-25, Nr. 5, Oktober 1977 (1977-10), Seiten 449-450, XP007901260 ISSN: 0096-3518
- "CLINICAL AUDIOMETERS IN REVIEW" HEARING INSTRUMENTS, HARCOURT BRACE JOVANOVICH PUBL. DULUTH, MINNESOTA, US, Bd. 40, Nr. 11, 1. November 1989 (1989-11-01), Seiten 38-40,42,44, XP000122782 ISSN: 0092-4466

## Beschreibung

Die Erfindung betrifft ein Verfahren zum Konfigurieren eines Hörgerätes für eine Person, bei dem ein Testtöne erzeugendes Audiometer und ein an das Audiometer angeschlossener, vom Patienten getragener, offener kopfhörer zum Übertragen von von dem Audiometer erzeugten Testtönen verwendet wird.

Menschen mit Gehörproblemen werden häufig mit Hörgeräten ausgerüstet, um die Hörprobleme aufzuheben bzw. zu lindern. Dabei ist es erforderlich, die Hörgeräte an die konkreten Anforderungen jedes Patienten anzupassen. Die Patienten weisen voneinander verschiedene Hörleiden auf, diese können sich auch in voneinander verschiedenen Stadien befinden. Die Hörgeräte sind an diese verschiedenen Leiden und verschiedenen Stadien anzupassen.

Um ein Hörgerät an einen Patienten anpassen zu können, ist es erforderlich, dessen Hörvermögen exakt zu ermitteln. Für dieses Ermitteln werden eingangs genannte Verfahren verwendet. Diese verwenden Audiometer oder objektive Meßgeräte, mit denen Testtöne erzeugt werden können, welche den Patienten zugeführt werden. Eine derartige Vorrichtung ist aus der US 2004/0204921 A1 bekannt.

Im Stand der Technik ist für das Zuführen der Testtöne ein genormter Kopfhörer vorgeschlagen worden. Dieser Kopfhörer umschließt das Ohr,
wobei ein genormtes geschlossenes Restvolumen von 20 cm³ verbleibt. Auf dieses Normmaß des Kopfhörers sind die bekannten Audiometer kalibriert. Problematisch ist aber, daß nicht bei jedem Patienten ein Restvolumen von 20 cm³ gegeben ist. Die Ohranatomien der Patienten sind verschieden. Aufgrund dieser Verschiedenheiten ist bei den Vorrichtungen nach dem Stand der Technik somit ein Fehler gegeben.

Auch wenn die Testtöne des Audiometers über einen auf dem Tisch stehenden Lautsprecher abgegeben werden, treten Fehler auf. Neben dem Ton aus dem Lautsprecher wird der Patient nämlich noch mit weiteren Geräuschquellen aus seiner Umgebung konfrontiert. Zudem nimmt das Testsignal auf dem Weg zwischen dem Lautsprecher und dem Patienten mit dem Quadrat der Entfernung ab.

Beim Auftreffen der aus dem geschlossenen Kopfhörer bzw. aus dem auf dem Tisch stehenden Lautsprecher austretenden Testtöne auf das Trommelfell des Patienten ist zudem noch dessen individuelle Steifigkeit zu berücksichtigen. Dadurch tritt ein weiterer Fehler auf.

Im Ergebnis ist die mit den bekannten Vorrichtungen durchgeführte Konfiguration des Hörgerätes ungenau. Die Konfiguration stellt sich als Regelkreis aus den Parametern geschlossener Kopfhörer, Hörgerät, Restvolumen des Gehörganges, Steifigkeit des Trommelfells, Ohreingangsresonanzen, Otoplastik (Ohrpaßstück) sowie periphere und zentrale Verarbeitung dar. Dieser Regelkreis ist nicht reproduzierbar. Das schwächste Glied in dieser Regelkreiskette bestimmt die Qualität des akustischen Signals. Allein auf die subjektiv empfundenen Auskünfte des Patienten wird die Konfiguration des Hörgerätes abgestellt. Das Empfinden des Patienten ist durch die genannten Fehler eingetrübt, der Patient wird häufig der Anpassungsprozedur überdrüssig sein und keine objektiven Auskünfte mehr geben. Er meldet nur noch ihm bekannte Höreindrücke und nicht Eindrücke, die zum Hören lernen notwendig sind, da natürliches Hören durch Entfremdung als normal gewertet wird.

Im Stand der Technik ist neben dem Einsatz von Hörgeräten auch der Einsatz von Implantaten vorgeschlagen worden. Diese Implantate können in den Ohrbereich eingesetzt werden, wenn das Hörvermögen absolut verloren gegangen ist. Die Implantate ersetzen die Hörzellen, der Träger eines derartigen Implantates vermag wieder hören und vermag auch ein Sprachverständnis zu entwickeln. Mit den Implantaten gelingt es dabei zunächst, die Hörschwelle des Patienten zu erreichen bzw. leicht zu überwinden.

Der Erfindung liegt die Aufgabe zugrunde, ein Verfahren der eingangs genannten Gattung aufzuzeigen, mit dem ein besseres Konfigurieren eines Hörgerätes durch Erreichen bzw. leichtes Überwinden der Hörschwelle des Patienten gelingt. Schwellennahe Testsignale sollen gleichfalls als Wahmehmungen garantiert werden, wie Signale angenehmen Hörens und der Unbehaglichkeitsschwelle.

Die verfahrensseitige Lösung der Aufgabe, für die selbständiger Schutz beansprucht wird, zeichnet sich dadurch aus, daß der Patient mit einem Hörgerät ausgestattet wird, daß am Audiometer voneinander verschiedene, feste Signalstärken eingestellt werden und daß eine Konfiguration des Hörgerätes in Abhängigkeit des individuellen Hörvermögens des Patienten durchgeführt wird, wobei mit dem Hörgerät das Signal des Audiometers bis zum Erreichen der Hörschwelle des Patienten frequenzspezifisch verstärkt wird.

Bei Verfahren zum Konfigurieren eines Hörgerätes wird im Stand der Technik nach der US 2004/0204921 A1 so vorgegangen, daß ein Audiometer verwendet wird, an dem variable Signalstärken eingestellt werden. Dieses variable Einstellen von lauten und leisen Signalen am Audiometer erfolgt nach dem erfindungsgemäßen Verfahren nicht. Vielmehr werden am Audiometer feste Signalstärken eingestellt, wobei z.B. kurzpulsige Signale fester Signalstärke, d.h. Lautstärke, verwendet werden. Diese Signale passieren zunächst das Hörgerät möglichst ohne eine Veränderung durch das Hörgerät, um die Hörschwelle des Patienten zu erreichen. Für den einzelnen Patienten wird dabei eine Signalstärke fest eingestellt, mit welcher seine Hörschwelle erreicht werden kann. Diese Signalstärke kann beispielsweise 0 dB oder auch 65 dB oder 90 dB bis zur Einstellgrenze betragen.

Die Erreichung der Hörschwelle des Patienten, erfolgt mit dem Hörgerät, beispielsweise an einem Desktop des Hörgerätes. Das Hörgerät kann dabei mit einer Steuereinrichtung, wie einem Personalcomputer, verbunden sein, um die optimale Konfiguration des Hörgerätes durchzuführen. Das Audiometer dient nunmehr lediglich als Signalquelle, die Audiometerfunktion wird durch den Desktop des Hörgerätes übernommen. Das als Signalquelle dienende Audiometer verfügt vorzugsweise über alle überschwelligen Tests, z.B. Langenbecksche Geräuschaudiometrie, SISSI-Test und Fowler-Test. Das Audiometer ist dabei vorzugsweise mehrkanalig, es kann zugleich bei Luftleitung und Knochenleitung mehrere Kanäle verschiedener Frequenzen und Pegel auch überschwellig zugleich anbieten. Das Audiometer ist dabei vorzugsweise zusätzlich mit einem Einsteckhörer in den Gehörgang des gegenüber dem Meßohrs liegende Ohr zu aktivierende Hörer ausgestattet, um Signale z.B. zur Verstärkung zu übermitteln

Bei dem erfindungsgemäßen Verfahren wird einerseits kein geschlossener Kopfhörer mit einem genormten Restvolumen verwendet. Damit ist dieser Fehler bereits ausgeschlossen. Es kommt aber auch kein entfernt vom Ohr positionierter offener Lautsprecher zum Einsatz, an dem viele Störgeräusche vorbeigelangen können. Es wird ein offener, kalibrierter Kopfhörer eingesetzt, um die Normfehler des geschlossenen Kopfhörers auszuschließen und um eine unmittelbare Einleitung der Testtöne in das Ohr zu gewährleisten.

Die eingesetzten Testtöne sind frei wählbar und entsprechen der Lautstärke eines Normalhörenden. Die Testtöne können alle möglichen Parameter aufweisen. Sie gelangen am Hörgerät vorbei, die Otoplastik passierend, in den Gehörgang des Patienten und werden über dessen periphere und zentrale Ohrorgane in das Gehirn eingeleitet. Ziel ist es, mit den Testtönen zunächst die Hörschwelle des Patienten zu ermitteln. Dazu wird mit dem Audiometer oder einem anderen Meßgerät z.B. ein Signal mit der Stärke der Hörschwelle des Patienten, wenn er gesund wäre, fest angeboten. Da der Patient ein Hörleiden hat, hört er zunächst nichts. Nun wird mit dem Hörgerät das Signal frequenzspezifisch weiter verstärkt, bis die Hörschwelle des Patienten neuronal erreicht ist. Er kann dann objektiv Auskunft darüber geben, wann dies eintritt. Anhand dieser objektiven Kenntnis ist es für den Hörgeräteakustiker möglich, das Hörgerät so zu konfigurieren, daß bei normalem Einsetzen des Hörgerätes die Hörschwelle des Patienten erreicht wird. Der Patient ist in der Lage, Schallwellen in seinen neuronalen Bereichen zu verarbeiten, so daß sich ein neuronales Netz neu ausbilden kann. Der Patient kann wieder hören lernen.

Bei dem erfindungsgemäßen Verfahren wird somit eine möglichst objektive Konfiguration des Hörgerätes vorgenommen. Die Parameter des Gerätes sowie bei der Konfiguration auftretende Fehlerquellen der Geräte nach dem Stand der Technik werden vermieden. Im Prinzip wird der Weg der Implantate beschritten, in der Weise, daß zunächst einmal die Hörschwelle des Patienten überhaupt objektiv erreicht wird.

Durch den Einsatz eines offenen Kopfhörers ist gewährleistet, daß der Lautsprecher dem Ohr des Patienten unmittelbar zugeordnet ist. Der Kopfhörer ist offen, so daß er die Gehörgänge des Patienten nicht verschließt. Vorzugsweise weist der Kopfhörer wie üblich zwei Lautsprecher auf, so daß beide Ohren des Patienten behandelt werden können, ohne ein Umsetzen des Kopfhörers zu benötigen.

Mit dem Hörgerät des Patienten kann eine Beeinflussung dieser Signale durchgeführt werden. Das Hörgerät funktioniert dann als Audiometer. Das Hörgerät weist dazu vorzugsweise einen insbesondere kalibrierten Desktop auf, über den es steuerbar bzw. programmierbar ist. Vorzugsweise können dabei alle Steuerungsfunktionen, über die das Audiometer in Bezug auf das Testsignal verfügt, auch bei dem desktopgesteuerten Hörgerät vorgesehen sein. Der Desktop ist frei programmierbar, seine Funktionen können mit den Funktionen des Audiometers verknüpft werden. So können bei Bedarf Audiometerfunktionen auch über den das Hörgerät programmierenden Desktop ausgeführt werden. Der Desktop des Hörgerätes kann dabei in das Hörgerät eingesetzt sein, er kann aber auch in einer externen und an das Hörgerät angeschlossenen Steuerungseinrichtung angeordnet sein. Diese Steuerungseinrichtung ist beispielsweise ein Personalcomputer. Mit dem Desktop des Hörgerätes können auch Signale für einen Knochenleitungshörer, der zu Meß- und/oder Vertäubungszwecken eingesetzt wird, erzeugt werden. Diese Vertäubungssignale werden als Rückschlüsse anhand der Nutzsignale gewonnen. Vertäubungssignale werden dabei über den Knochenschall oder Luftschall oder zugleich auch mit zeitlichen, pegelabhängigem Frequenzversatz bzw. entsprechenden Automatikfunktionen übertragen.

Die gewählten Testtöne des Audiometers können Shirp-Signale, Speechmap-Signale oder Schmalband-Signale sein. Der Kopfhörer kann zusätzlich wenigstens einen Knochenleitungshörer aufweisen. Dieser kann dazu eingesetzt werden, ein Ohr, insbesondere ein besser hörendes, zu vertäuben. Das eingesetzte Audiometer kann auch langwellige bzw. langpulsige Signale, wie beispielsweise Sinussignale einsetzen. Zum Auffinden der Hörschwelle des Patienten werden aber die in der Regel kurzpulsigen Signale erfindungsgemäß eingesetzt.

Zur Kontrolle der Arbeitsweise des Desktop-gesteuerten Hörgerätes kann eine im Gehörgang eines Patienten anordbare In-Situ-Sonde an eine Meßeinrichtung angeschlossen sein und auf diese Weise wird überprüft, ob der als Audiometer eingesetzte Desktop des Hörgerätes auch die gewünschten Schwellwerte erreicht. Das Ergebnis der Kontrolle kann optisch oder akustisch angezeigt werden. Die In-Situ-Sonde ist dabei am Eingang des Gehörganges eines Patienten angeordnet. Neben der vorgenannten Kontrolle kann diese Sonde durch ihre Verschaltung mit einer Meßeinrichtung auch überprüfen, ob der richtige Schalldruckpeckel des Kopfhörers gegeben ist. Mit Hilfe der Meßeinrichtung können dazu Substitutions- und/oder Komparationsverfahren angewandt werden. Statt einer In-Situ-Sonde kann auch ein Computertomograph eingesetzt werden, dessen Ergebnisse für die Überwachung eingesetzt werden können und zur Programmierung über den kalibrierten Desktop dem Hörgerät dienen.

Bei einem Messverfahren kann dann so vorgegangen werden, daß tiefe Frequenzen nach Wahl in Gruppen zusammengefaßt werden und Pegel zusammenfassend im Bereich des Störlärms ein Signal abgeben, welches so intensiv ist, daß Störgeräusche oberhalb der niederfrequenten Hörschwelle des zu messenden Betroffenden, also der umgebende Störschall (hauptsächlich niederfrequenter Art), überschwellig vedeckt wird, ohne eine Aufwärtsmaskierung der zu messenden Hochfrequenzanteile zu provozieren (ähnlich des Carhardt-Testes oder Langenbeckschen Geräuschaudiometrie). Für Innenohrgeschädigte ist es z.B. beim SISSI-Test erheblicher einfacher, Incremente mit 1dB oder mehr Intensitätserhöhung herauszuhören, als Normalhörige. Trotz Anbietung eines Störschalls oberhalb der Hörschwelle in diesem Bereich zur Auslöschung von Umgebungsgeräuschen, ist das Messen hochfrequenter, energiearmer aber informationsreicher Schwellenwerte zur Sprachanbahnung von Konsonanten weiter meßbar, wenn diese lauter sind als die Vertäubungsgeräusche

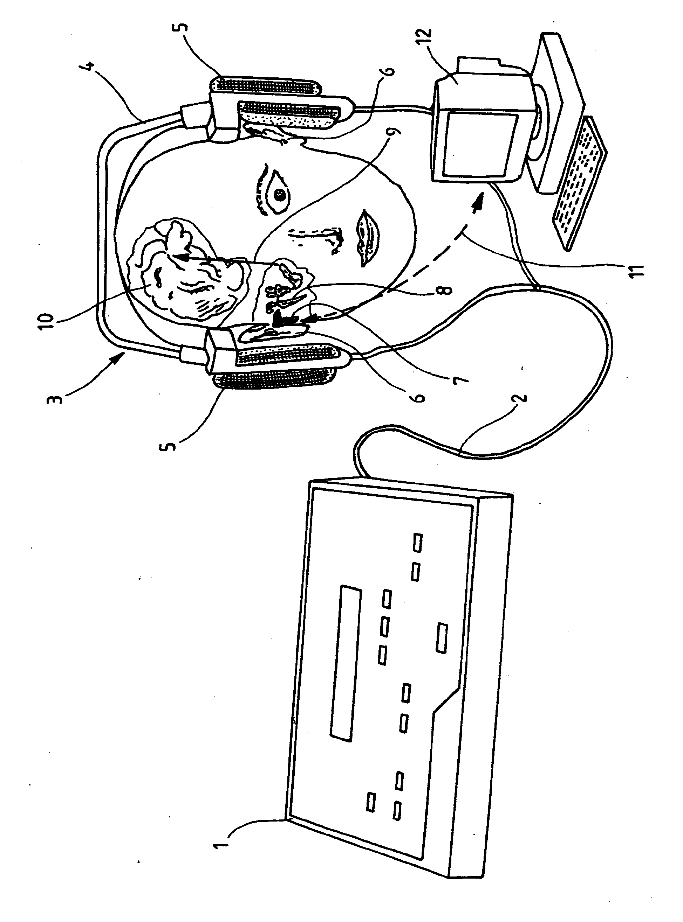

Ein Ausführungsbeispiel, aus dem sich weitere erfinderische Merkmale ergeben, ist in der Zeichnung dargestellt. Die einzige Figur der Zeichnung zeigt in schematischer Weise eine erfindungsgemäße Vorrichtung zum Konfigurieren eines Hörgerätes.

Die Vorrichtung in der Figur weist ein Audiometer 1 auf. Das Audiometer 1 kann voneinander verschiedene akustische Signale erzeugen. Diese Signale werden über eine Kabelverbindung 2 auf einen Kopfhörer 3 gegeben.

Bei dem Kopfhörer 3 handelt es sich um einen offenen Kopfhörer. An einem Gestell 4 sind zwei offene Lautsprecher 5 angeordnet, wobei jeder Lautsprecher 5 einem Ohr 6 des Patienten zugeordnet ist.

In das rechte Ohr 6 des Patienten ist ein Hörgerät 7 eingesetzt. Das eingeschaltete Hörgerät 7 ist im Gehörgang des rechten Ohres 6 angeordnet, es steht mit den inneren Organen 8 des Ohrs 6 in unmittelbarem Kontakt. Ausgehend von den inneren Organen 8 erfolgt eine mit einem Pfeil 9 angedeutete Reizleitung zum Gehirn 10 des Patienten.

Bei dem Beaufschlagen des Kopfhörers 3 mit kurzpulsigen Testtönen durch das Audiometer 1 passieren diese Testtöne den Gehörgang mit dem Hörgerät 7 ohne eine Veränderung durch das Hörgerät 7. Die inneren Organe 8 werden angeregt und es erfolgt bei Überschreiten der Hörschwelle eine Reizleitung entlang des Pfeils 9 zum Gehirn 10. Der Patient kann eine Rückmeldung geben. Das Hörgerät 7 wird nun über eine durch den gestrichelten Pfeil 11 angedeutete Verbindung mit einer Einstellanlage 12 derart konfiguriert, daß bei dem Auftreffen normaler akustischer Signale eine Anregung des Gehirns 10 erfolgt und somit das Hörempfinden des Patienten verbessert wird.

## Patentansprüche

1. Verfahren zum Konfigurieren eines Hörgerätes für eine Person, bei dem ein Testtöne erzeugendes Audiometer und ein an das Audiometer angeschlossener, vom Patienten getragener offener Kopfhörer zum Übertragen von von dem Audiometer erzeugten Testtönen verwendet wird,
wobei der Patient mit einem Hörgerät ausgestattet wird, am Audiometer voneinander verschiedene, feste Signalstärken eingestellt werden und eine Konfiguration des Hörgerätes in Abhängigkeit des individuellen Hörvermögens des Patienten durchgeführt wird, , **dadurch gekennzeichnet, dass** mit dem Hörgerät das Signal des Audiometers bis zum Erreichen der Hörschwelle des Patienten Frequenzspezifisch verstärkt wird.

2. Verfahren nach Anspruch 1, **dadurch gekennzeichnet, dass** am Audiometer eine Signalstärke von etwa 0 dB eingestellt wird.

3. Verfahren nach Anspruch 1 oder 2, **dadurch gekennzeichnet, dass** am Audiometer eine Signalstärke von etwa 65 dB eingestellt wird.

4. Verfahren nach einem der vorhergehenden Ansprüche, **dadurch gekennzeichnet, dass** am Audiometer eine Signalstärke von über etwa 90 dB bis zur Einstellgrenze eingestellt wird.

5. Verfahren nach einem der Ansprüche 1 bis 4, **dadurch gekennzeichnet, dass** das Hörgerät über einen Desktop gesteuert wird.

## Claims

1. A method for configuring a hearing aid for a person, in which an audiometer generating test sounds and an open earphone which is connected to the audiometer and worn by the patient, is used for transmitting test sounds generated by the audiometer, wherein the patient is fitted with a hearing aid, fixed signal strengths which differ from one another are set on the audiometer and the hearing aid is configured as a function of the individual hearing capacity of the patient, **characterised in that** the signal of the audiometer is amplified in a frequency-specific manner with the hearing aid until the hearing threshold of the patient is reached.

2. The method according to claim 1, **characterised in that** a signal strength of about 0 dB is set on the audiometer.

3. The method according to claim 1 or 2, **characterised in that** a signal strength of about 65 dB is set on the audiometer.

4. The method according to any one of the preceding claims, **characterised in that** a signal strength of above about 90 dB is set on the audiometer until the setting limit is reached.

5. The method according to any one of claims 1 to 4, **characterised in that** the hearing aid is controlled by means of a desktop computer.

## Revendications

1. Procédé de configuration d'un appareil auditif destiné à une personne, dans lequel un audiomètre produisant des tonalités de test et un casque d'écoute ouvert porté par le patient, raccordé à l'audiomètre est utilisé pour transmettre des tonalités de test produites par l'audiomètre, moyennant quoi le patient est équipé d'un appareil auditif, des intensités de signal fixes, différentes les unes des autres sont réglées sur l'audiomètre et une configuration de l'appareil auditif est effectuée en fonction de la capacité auditive individuelle du patient, **caractérisé en ce que** le signal de l'audiomètre est amplifié à une fréquence spécifique avec l'appareil auditif jusqu'à ce que le seuil auditif du patient soit atteint.

2. Procédé selon la revendication 1, **caractérisé en ce que** une intensité de signal d'environ 0 dB est réglée sur l'audiomètre.

3. Procédé selon la revendication 1 ou 2, **caractérisé en ce que** une intensité de signal d'environ 65 dB est réglée sur l'audiomètre.

4. Procédé selon une des revendications précédentes, **caractérisé en ce que** une intensité de signal allant d'une valeur quelque peu supérieure à 90 dB jusqu'à la valeur de réglage est réglée sur l'audiomètre.

5. Procédé selon une des revendications 1 à 4,
**caractérisé en ce que** l'appareil auditif est commandé par l'intermédiaire d'un bureau électronique.
